# EUROPEAN PATENT APPLICATION

(11) **EP 4 332 843 A1**
(43) Date of publication of application: **06.03.2024**
(21) Application number: 22382805.4
(22) Date of filing: 29.08.2022
(51) Int. Cl.: G06N 10/60, G06N 3/042, G06N 20/10, G06N 3/045, G06N 20/20, G06F 3/01, A61B 5/369, G01R 33/26

(54) **BRAIN ACTIVITY SENSING AND COMPUTER INTERFACING**

(71) Applicant: Multiverse Computing S.L., 20014 Donostia-San Sebastian (ES)
(72) Inventor: Orús, Román, Donostia-San Sebastian (ES); Sheikh, Usman, Donostia-San Sebastian (ES)
(74) Representative: Balder IP Law, S.L.

(57) **Abstract**

A method comprising: receiving, by at least one classical computing device or at least one quantum computing device, brain activity measurements of a person provided by at least one brain activity sensor; and processing, by the at least one classical computing device or the at least one quantum computing device, the brain activity measurements by inputting the measurements into a machine learning pipeline configured to provide data indicative of commands or thoughts of the person's brain, the machine learning pipeline being trained with a training dataset with brain activity measurements of the person and commands or thoughts associated therewith. Also, a system for brain activity sensing and computer interfacing.

## Description

### TECHNICAL FIELD

The present disclosure relates to the technical field of sensors. More particularly, the present disclosure relates to brain activity sensors and brain computer interfaces.

### BACKGROUND

Brain computer interfaces (BCI) are capable of increasing quality of life by either enhancing or substituting the human peripheral working capacity. This is even more so in people having a medical condition such that they have more limited capacity of action, for instance, limited hand or arm activity, limited leg activity, etc.

BCIs rely on brain activity sensors that measure brain signals (e.g. electroencephalogram, magnetic resonance images, magnetoencephalogram etc.). The BCIs process the brain signals to decode what are the thoughts or intentions of the person, and derive an output associated with those thoughts or intentions, like commands for actuation of electronic devices whose operation can be dynamically or automatically modified by providing them with the proper commands.

A common problem that BCIs have is that brain signals are complex to decode, hence the resulting outputs are oftentimes incorrect. Among the different types of brain signals, the ones simpler to decode, yet complex anyway, correspond to cognitive processes that are conscious since they are relatively less noisy, general and isolated from interfering processes. This type of brain signals is, more or less successfully, decoded in existing BCIs, but more complex brain signals are not, or at least the outputs of the BCIs typically do not correspond to the actual thoughts or intentions of the person. A reason for the decoding complexity of brain signals is that it is considered that the human brain is reducible to a classical computer, yet most mental processes do not follow the algorithmic approach/workflow in the classical context and thus cannot be reduced to classical computation processes.

Methods, devices and systems that overcome the limitations of existing BCIs are sought.

### SUMMARY

A first aspect of the disclosure relates to a method comprising:
receiving, by at least one classical computing device or at least one quantum computing device, brain activity measurements of a person provided by at least one brain activity sensor; and
processing, by the at least one classical computing device or the at least one quantum computing device, the brain activity measurements by inputting the measurements into a machine learning pipeline configured to provide data indicative of commands or thoughts of the person's brain, the machine learning pipeline being trained with a training dataset with brain activity measurements of the person and commands or thoughts associated therewith.

The brain activity of the person is processed with the machine learning pipeline that establishes relationships between the different measurements of the brain activity of the person and the actual commands or thoughts so that outputs of the pipeline may corresponds to said actual commands or thoughts. Owing to machine learning, as new data comes in the pipeline modifies the relationships on its own to increase the effectivity in deriving the commands or thoughts.

Machine learning pipelines enable meaningful processing of brain activity measurements that can be noisy and unreliable due to the nature of the brain activity and the limitations of the sensors themselves. By way of example, electroencephalogram measurement signals, which are one possibly type of brain activity measurements, feature high temporal resolution but low spatial resolution, which in turn make difficult the determination of what the person thought. Hence, machine learning makes possible to distinguish between different kinds of mental states.

The machine learning pipeline preferably at least comprises feature extraction processing of the brain activity measurements and classification processing of the measurements and the features to classify the fresh measurements into different types of outputs, e.g. a first predetermined command, a second predetermined command, a first predetermined thought, etc.

The machine learning pipeline is trained with data associated with the same person to more accurately derive the outputs based on fresh brain activity measurements.

In some embodiments, the method further comprises commanding, by the at least one classical computing device when an output of the machine learning pipeline is at least one command, at least one electronic device to run the at least one command to control operation thereof or to control, a first electronic device of the at least one electronic device, operation of a second electronic device of the at least one electronic device.

Once the machine learning pipeline determines the command or thought, the pipeline can provide it for actuation of devices or for information purposes.

To this end, the at least one classical computing device may process the output of the machine learning pipeline to associate it with one or more tasks that correspond to a command, and then provide or transmit said command with one or more tasks for changing the operation of any given device. Alternatively, the command as outputted by the pipeline may be provided as is or in some processed form, and the electronic device that is to run the command determine which task or tasks have to be run.

By way of example, when the outputted command is turn on the air conditioner, a set of tasks may be associated with such command like: check if the windows and doors are closed, turn on the air conditioner if windows and doors are closed, check the temperature inside the room, and change the temperature of the air conditioner to be 3 degrees below the room temperature. By way of another example, an outputted thought can be e.g. things that the person may want to say or write but is unable to due to some pathology, movements that the person may want to do but cannot, etc.; these thoughts can be converted into tasks of a command like, for instance, make a synthetic voice say the thoughts through loudspeakers, etc. These tasks are then provided in the form of a command for actuation, either by the at least one classical computing device or by the at least one electronic device.

In some embodiments, the at least one electronic device is arranged on the person. In some other embodiments, the at least one electronic device is remote from the person.

One or more electronic devices can be, for instance, an exosuit, a robotic arm, a robotic leg, etc. Alternatively, one or more electronic devices may be, for instance, a home automation controller, means for transportation such as cars, a personal computer, loudspeakers, etc.

In some embodiments, the processing is conducted by the at least one classical computing device, and the machine learning pipeline is a classical machine learning pipeline.

The processing power of classical computing devices is generally more limited than that of quantum computing devices, and even if the sensor(s) used for brain activity measurements has limited resolution, frequency, etc. in the measurements thereof, classical machine learning pipelines are capable of providing an effective brain activity sensor and computer interface.

In these cases, the brain activity measurements may be received by the at least one classical computing device, or by the at least one quantum computing device, which may perform some processing on it or not before passing it onto the at least one classical computing device for processing according to the machine learning pipeline.

In some embodiments, the classical machine learning pipeline comprises one of the following: k-nearest neighbors, a support vector machine, a neural network, and an ensemble method-based algorithm.

In the k-nearest neighbors' strategy, the data is clustered according to the distances amongst the datapoints in a mathematical space. In a support vector machine, the method finds a hyperplane that separates different classes of datapoints, corresponding to different interpretations of the brain signals. In the neural network and ensemble method algorithm, the data is classified by training a set of parameters in the algorithm in order to minimize the error in the classification, with every class corresponding to a different interpretation of a brain signal.

In some embodiments, the classical machine learning pipeline comprises one of the following: a tensor network support vector machine, a tensor neural network, and a tensor convolutional neural network.

Quantum-inspired machine learning pipelines that rely on tensors and tensor networks provide superior capacity in the processing of brain activity measurements.

In some embodiments, the at least one classical computing device comprises one or more of: a central processing unit, CPU, a graphics processing unit, GPU, a field-programmable gate array, FPGA, and an application-specific integrated circuit, ASIC.

In some embodiments, the processing is conducted by the at least one quantum computing device, and the machine learning pipeline is a quantum machine learning pipeline.

The processing power of quantum computing devices increases the complexity of the machine learning pipelines to be processed in a short time span. BCIs require low reaction times, namely the latency in providing a response after the brain activity measurement must be low, otherwise they are ineffective BCIs for many applications. The more complex the pipeline becomes, the better the output it might provide but at the same time the longer time it takes to provide the output, yet with quantum computing devices this problem is reduced.

Further, when the at least one sensor comprises quantum sensors, which have a larger bandwidth in terms of data they provide, classical computing devices are usually not capable of processing such large amounts of data, let alone in a short time span, thus making classical computing devices unsuitable for quantum sensors.

In these cases, the brain activity measurements may be received by the at least one quantum computing device, or by the at least one classical computing device, which may perform some processing on it or not before passing it onto the at least one quantum computing device for processing according to the machine learning pipeline.

In some embodiments, the at least one quantum computing device comprises a quantum platform based on one of the following: superconducting circuits, trapped ions, neutral atoms, and photonics.

In some embodiments, the quantum machine learning pipeline comprises one of the following: a quantum support vector machine, a quantum neural network, a quantum ensemble method-based algorithm, a quantum boost algorithm, and a variational quantum circuit with data reuploading.

In the quantum support vector machine, the data of the patients is used to determine a mathematical hyperplane that separates patients with different characteristics (e.g., risky vs non-risky). In the variational quantum classifier with data reuploading, the parameters in a quantum circuit are fine-tuned to minimize the error in the classification, similarly to quantum neural networks. In the algorithms based on quantum boost, an ensemble of weak classifiers (which could be classical) are combined with weights, so that the weights are optimized in order to minimize the overall error in the classification of the patients. All these mathematical procedures can be specifically adapted to the number of features in the datapoints, which include the brain signals being taken from the subjects.

In addition to the solving capabilities of quantum machine learning pipelines, these pipelines also feature speedier training thereof, thereby reducing the time it takes the BCI to become fully operative.

In some embodiments, the at least one brain activity sensor measures the brain activity with at least one of the following: an electroencephalogram, magnetic resonance images, and a magnetoencephalogram.

In some embodiments, the at least one brain activity sensor comprises at least one quantum brain activity sensor.

In some embodiments, the at least one quantum brain activity sensor comprises one or both of: at least one optically pumped magnetometer, and a diamond magnetometer.

These quantum magnetometers provide more data than some classical sensors, for example traditional electroencephalograms. Particularly, this type of magnetometers features richer spatial and temporal resolution. Given the complexity of brain activity decoding, additional data for processing and deriving the brain activity is important for an effective BCI.

In some embodiments, the method further comprises measuring the brain activity of the person with the at least one brain activity sensor.

In some embodiments, the method further comprises training, by the at least one classical computing device or the at least one quantum computing device, the machine learning pipeline with the training dataset.

For the training, the at least one brain activity sensor likewise measures the brain activity and provides the measurements for processing them into the machine learning pipeline. As part of the training dataset, preferably the person inputs data corresponding to the commands or thoughts the person had for the training to be more effective. To this end, the person can input such data via one or more user input devices (e.g. microphone, keyboard, touchscreen, etc.).

A second aspect of the disclosure relates to a device or system comprising: means adapted to execute the steps of a method according to the first aspect.

The device or system is a brain activity sensor and, optionally, a brain computer interface capable of processing brain activity measurements of people in a manner more reliable than known sensors and BCIs.

A third aspect of the disclosure relates to a system comprising:
at least one brain activity sensor; and
at least one classical computing device or at least one quantum computing device, the device being configured to execute the steps of a method according to the first aspect.

In some embodiments, the system comprises both the at least one classical computing device and the at least one quantum computing device.

In some embodiments, the at least one brain activity sensor comprises the at least one quantum brain activity sensor.

In some embodiments, the system further comprises at least one electronic device configured to receive one or more commands from the at least one classical computing device. In some embodiments, the at least one electronic device is configured to run the one or more commands for changing operation of the at least one electronic device. In some embodiments, the at least one electronic device at least comprises a first electronic device and a second electronic device, the first electronic device being configured to receive the one or more commands and run the one or more commands for changing operation of the second electronic device.

A fourth aspect of the disclosure relates to a computer program product that has instructions which, when executed by at least one classical computing device, cause the at least one classical computing device to carry out the steps of a method according to the first aspect.

In some embodiments, the computer program product is embodied on a non-transitory computer-readable medium or a computer-readable data carrier has the computer program product stored thereon.

A fifth aspect of the disclosure relates to a data carrier signal carrying a computer program product according to the fourth aspect.

Similar advantages as those set out with reference to the first aspect of the disclosure are likewise applicable to the remaining aspects of the disclosure.

### BRIEF DESCRIPTION OF THE DRAWINGS

To complete the description and in order to provide for a better understanding of the disclosure, a set of drawings is provided. Said drawings form an integral part of the description and illustrate embodiments of the disclosure, which should not be interpreted as restricting the scope of the disclosure, but just as examples of how the disclosure can be carried out. The drawings comprise the following figures:
Figure 1 shows a system in accordance with some embodiments.
Figure 2 shows a method in accordance with some embodiments.

### DETAILED DESCRIPTION

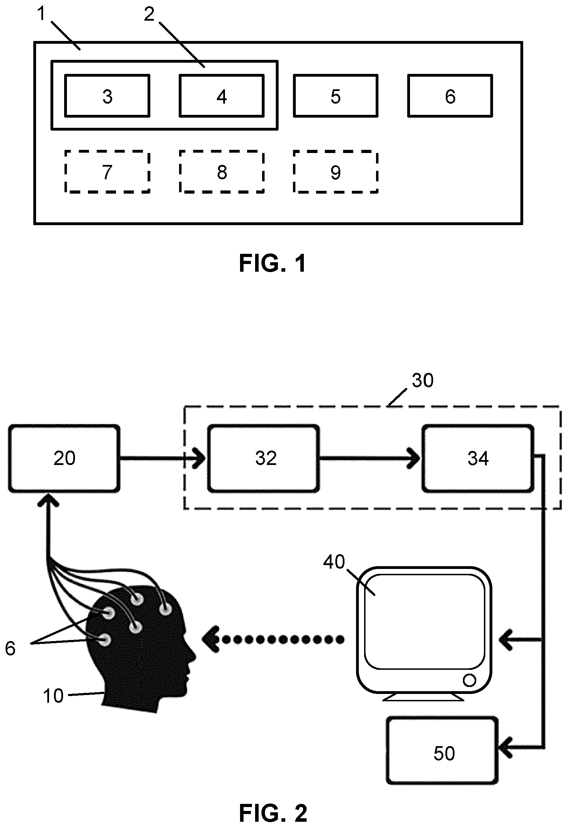

Figure 1 shows a system 1 for brain activity sensing and computer interfacing in accordance with some embodiments.

The system 1 comprises one or more classical computing devices 2 and/or one or more quantum computing devices 5. Methods according to the present disclosure can be carried out by the system 1.

Each of the classical computing devices 2 comprises at least one processor 3 and at least one memory 4 for running a machine learning pipeline, namely a machine learning program or algorithm, for brain activity computer interfacing. Classical computing devices 2 may be, for example, a CPU, a GPU, an FPGA, an ASIC, a personal computer, a laptop, etc. The at least one memory 4 may include instructions, for example in the form of a computer program code, so that a method according to the present disclosure is carried out upon execution by the at least one processor 3.

The one or more quantum computing devices 5 may also be for running a machine learning pipeline for brain activity computer interfacing.

The system 1 also comprises at least one brain activity sensor 6 configured to measure brain signals of a person. The at least one sensor 6 may have to be in contact with the person like, for instance, when the measurements are an electroencephalogram, EEG, but it may also not have to be in contact with the person like, for instance, when the measurements are magnetic resonance images, MRI. In some embodiments, the at least one sensor 6 has one or more quantum sensors like e.g. optically pumped magnetometers and/or diamond magnetometers.

As shown with dashed lines for the sake of clarity only, the system 1 may also comprise one or more of the following: a communications module 7 for wired or wireless communications between the one or more classical computing devices 2 and some other devices, including the one or more quantum computing devices 5, one or more user presenting devices 8, and one or more user input devices 9. The communications module 7 might include a converter of signals for the one or more classical computing devices 2 into signals for the one or more quantum computing devices 5 and vice versa, even though, sometimes, the quantum computing device(s) 5 already includes such a converter.

Figure 2 shows a method in accordance with some embodiments.

A person 10 has its brain activity sensed by way of one or more brain activity sensors 6. The measurements of the person's brain are preferably amplified and, then, digitized in a stage 20 adapted to that end. One or more electronic circuits and analog-to-digital converters can be arranged in the amplification and digitization stage 20.

The digitized measurements are then provided to one or more classical computing devices and/or one or more quantum computing devices configured to control and, possibly, run a machine learning pipeline 30 that is to process the digitized measurements and provide, in the form of one or more outputs, commands or thoughts the person 10 had when the brain activity thereof was measured by the sensor(s) 6.

The machine learning pipeline 30 may be a classical machine learning pipeline, in which case the one or more classical computing devices run it, or a quantum machine learning pipeline, in which case one or more quantum computing devices run it. In the latter case, the one or more classical computing devices may optionally provide the digitized measurements to the quantum computing device(s) to use them in the machine learning pipeline; in some embodiments, prior to the provision of the measurements to the quantum computing device(s), the classical computing device(s) process the digitized measurements for e.g. data cleaning and/or data preprocessing.

In the machine learning pipeline 30, the measurements are processed in a feature extraction stage 32 whereby features within the data are gathered as known in the art of machine learning. Then, the measurements and the features thereof are passed onto a classification stage 34, according to a procedure known in the art of machine learning, configured to process the data to determine the commands or thoughts of the person 10. To this end, the classification stage 34 is previously trained with brain activity measurements of the person 10 and data manually inputted thereto corresponding to the commands and thoughts the person 10 had. Given the heterogeneity of human brains, the training is preferably conducted with a training dataset for the same person 10 whose brain activity will then have to be processed with the machine learning pipeline 30, thereby increasing the accuracy with which the pipeline 30 derives commands or thoughts.

Outputs of the machine learning pipeline 30 are, in some embodiments, provided to e.g. a user presenting device 40 to produce a feedback loop with the person 10, especially during training of the machine learning pipeline 30 so that the person 10 is able to set whether the outputs are correct or wrong.

The outputs of the pipeline 30 are processed by the at least one classical computing device in an output processing stage 50 to provide a command associated with the outputs that can be used to control itself or one or more electronic devices. The provided command may be run by the at least one classical computing device or be transmitted to the one or more electronic devices which, in turn, will run the command for actuating one or more parts thereof or of other devices.

In this text, the term "includes", "comprises" and its derivations (such as "including", "comprising", etc.) should not be understood in an excluding sense, that is, these terms should not be interpreted as excluding the possibility that what is described and defined may include further elements, steps, etc.

On the other hand, the disclosure is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

## Claims

1. A method comprising:
receiving, by at least one classical computing device or at least one quantum computing device, brain activity measurements of a person provided by at least one brain activity sensor; and
processing, by the at least one classical computing device or the at least one quantum computing device, the brain activity measurements by inputting the measurements into a machine learning pipeline configured to provide data indicative of commands or thoughts of the person's brain, the machine learning pipeline being trained with a training dataset with brain activity measurements of the person and commands or thoughts associated therewith.

2. The method of claim 1, further comprising: commanding, by the at least one classical computing device when an output of the machine learning pipeline is at least one command, at least one electronic device to run the at least one command to control operation thereof or to control, a first electronic device of the at least one electronic device, operation of a second electronic device of the at least one electronic device.

3. The method of any one of the preceding claims, wherein the processing is conducted by the at least one classical computing device, and the machine learning pipeline is a classical machine learning pipeline.

4. The method of claim 3, wherein the classical machine learning pipeline comprises one of the following: k-nearest neighbors, a support vector machine, a neural network, and an ensemble method-based algorithm.

5. The method of claim 3, wherein the classical machine learning pipeline comprises one of the following: a tensor network support vector machine, a tensor neural network, and a tensor convolutional neural network.

6. The method of any one of claims 1-2, wherein the processing is conducted by the at least one quantum computing device, and the machine learning pipeline is a quantum machine learning pipeline.

7. The method of claim 6, wherein the quantum machine learning pipeline comprises one of the following: a quantum support vector machine, a quantum neural network, and a quantum ensemble method-based algorithm.

8. The method of any one of the preceding claims, wherein the at least one brain activity sensor measures the brain activity with at least one of the following: an electroencephalogram, magnetic resonance images, and a magnetoencephalogram.

9. The method of any one of claims 1-7, wherein the at least one brain activity sensor comprises at least one quantum brain activity sensor.

10. The method of claim 9, wherein the at least one quantum brain activity sensor comprises one or both of: at least one optically pumped magnetometer, and a diamond magnetometer.

11. The method of any one of the preceding claims, further comprising measuring the brain activity of the person with the at least one brain activity sensor.

12. A device or system comprising means adapted to execute the steps of a method according to any one of the preceding claims.

13. A system comprising:
at least one brain activity sensor; and
at least one classical computing device or at least one quantum computing device, the device being configured to execute the steps of a method according to any one of claims 1-11.

14. The system of claim 13, wherein the system comprises both the at least one classical computing device and the at least one quantum computing device.

15. A computer program product that has instructions which, when the program is executed by at least one classical computing device, cause the at least one classical computing device to carry out the steps of a method according to any one of claims 1-11.
